# EUROPEAN PATENT APPLICATION

(11) **EP 1 369 109 A1**
(43) Date of publication of application: **10.12.2003**
(21) Application number: 02705081.4
(22) Date of filing: 05.03.2002
(51) Int. Cl.: A61K 9/20, A61K 47/10, A61K 47/32, A61K 47/36, A61K 47/38

(54) **PREPARATIONS QUICKLY DISINTEGRATING IN ORAL CAVITY**

(30) Priority: 06.03.2001 JP 2001062692
(71) Applicant: KYOWA HAKKO KOGYO CO., LTD., Chiyoda-ku, Tokyo 100-8185 (JP)
(72) Inventor: NARITA, Shoichi, c/o Kyowa Hakko Kogyo Co., Ltd, Sunto-gun, Shizuoka 411-8731 (JP); OUCHI, Kazue, c/o Kyowa Hakko Kogyo Co., Ltd, Sunto-gun, Shizuoka 411-8731 (JP); MIYABE, Junichi, c/o Kyowa Hakko Kogyo Co., Ltd, Sunto-gun, Shizuoka 411-8731 (JP); MURAI, Kouji, c/o Kyowa Hakko Kogyo Co., Ltd, Sunto-gun, Shizuoka 411-8731 (JP); OGASA, Takehiro, c/o Kyowa Hakko Kogyo Co., Ltd, Tokyo 100-8185 (JP); OHTA, Motohiro, c/o Kyowa Hakko Kogyo Co., Ltd, Sunto-gun, Shizuoka 411-8731 (JP)
(74) Representative: Best, Michael, Dr.
(86) International application number: JP0202049
(87) International publication number: WO02069934

(57) **Abstract**

In accordance with the present invention, there is provided an intraorally rapidly disintegrable preparation which comprises a spray-dried powder comprising a sugar alcohol, of which unit particle is a primary particle, and an active ingredient, which has hardness having no problem in practical use and shows good disintegrability in the oral cavity. The above preparation can be produced by a common compression molding method, preferably by a direct tableting method.

## Description

### Technical Field

The present invention relates to a preparation which disintegrates rapidly in the oral cavity.

### Background of the Invention

A sugar alcohol has been widely used as an additive in the fields of foods, pharmaceuticals and feed. From the results in actual use up to now, it has been disclosed that a sugar alcohol is an additive having high safety and has good chemical and biological stabilities. In recent years, preparations which easily disintegrate or dissolve in the oral cavity (intraorally rapidly disintegrable preparations) making use of high water solubility of a sugar alcohol have been invented and actually used, and they are receiving public attention as preparations for patients having weak deglutition such as aged or infant patients.

However, a sugar alcohol has poor binding property in compression molding and is inferior in compression-moldability and, therefore, it has been rare that an intraorally rapidly disintegrable preparation comprising a sugar alcohol is made into compression-molded preparations such as tablets.

Under such circumstances, various investigations for making intraorally rapidly disintegrable preparations comprising a sugar alcohol into compression-molded preparations such as tablets have been carried out and, as a result, there has been developed a method for the manufacture of intraorally rapidly disintegrable preparations comprising a sugar alcohol by the use of a special compression molding operation. An example of such a method is a method where a mixture comprising a drug, a water-soluble binder and a water-soluble excipient such as a sugar alcohol is made into tablets at low pressure, then moisturized and dried (Japanese Patent No. 2,919,771). In the method, however, moisturization and drying have to be carried out after tableting, thus there are problems that a lot of steps are required in the manufacturing process and that its application to drugs which are unstable in water is difficult. There have been also known an intraorally rapidly disintegrable preparation prepared by a combination of a water-soluble excipient such as a sugar alcohol with an amorphous saccharide, and a method for manufacturing the same (Japanese Published Unexamined Patent Application No. 12161/1999). In the method, however, enhancement of hardness by aging is essential and the problem of securing the place for preservation during the period and the problem of becoming the manufacturing period longer are unavoidable, whereby the manufacturing method has no good manufacturing efficiency. Thus, even when a sugar alcohol is used as a main additive to an intraorally rapidly disintegrable preparation, there has been still a demand for development of an art which would make the manufacture by a common compression molding possible.

Japanese Published Unexamined Patent Application No. 43429/1999 and WO 97/47287 disclose compression-molded preparations which disintegrate rapidly in the oral cavity comprising a sugar alcohol, have good compression-moldability and can be manufactured by common compression molding methods. However, in those publications, the use of powder comprising a sugar alcohol obtained by spray-drying is not described. Further, in the inventions mentioned in the above publications, there is used a manufacturing method which is a so-called indirect tableting method where granules comprising a sugar alcohol are once prepared before compression molding and then other components such as a lubricant are added thereto followed by tableting, and there is no description concerning a direct tableting method including no granulation step. A direct tableting method is easier and simpler than an indirect tableting method. In addition, since there are some cases where granulation is difficult in view of stability and the like, there has been a wide demand for making the manufacture by a direct tableting possible not only in the case of preparations disintegrating quickly in the oral cavity but also in the manufacture of tablets as a whole. Further, in the above inventions, it is necessary to mix other additives, particularly a disintegrant agent other than a sugar alcohol, for achieving sufficient moldability and disintegrability.

On the other hand, use of saccharide powders or sugar alcohol powders obtained by spray-drying in compression-molded preparations is described, for example, in Japanese Patent No. 2,874,778 and Japanese Published Unexamined Patent Application No. 85330/1986. However, there is no description in the publications about the use of powders of a saccharide or a sugar alcohol obtained by spray-drying in intraorally rapidly disintegrable preparations. Accordingly, in order to use powders of a saccharide or a sugar alcohol obtained by spray-drying as an additive for intraorally rapidly disintegrable preparations, further improvements have been required to enhance moldability and disintegrability of the preparations.

From the above, there has not been disclosed any art concerning intraorally rapidly disintegrable preparations using spray-dried powder comprising a sugar alcohol as an additive, and also there has not been disclosed any art for manufacturing intraorally rapidly disintegrable preparations by means of a direct tableting method. Thus, such art has not been known.

### Disclosure of the Invention

An object of the present invention is making it possible to manufacture a preparation disintegrating quickly in the oral cavity having good disintegrability and compression-moldability by a common compression molding or preferably by a direct tableting.

Another object of the present invention is to provide a preparation disintegrating quickly in the oral cavity which has hardness having no problem in practical use, has good disintegrability in the oral cavity and preferably shows good disintegrability even in the case where no disintegrant other than the spray-dried powder comprising a sugar alcohol is specially added thereto.

The present inventors have carried out intensive investigations and found that, when spray-dried powder comprising a sugar alcohol of which unit particle is primary particle is used, it is now possible to manufacture a preparation disintegrating quickly in the oral cavity by means of a common compression molding or preferably by a direct tableting by the use of a tableting machine having high productivity such as a rotary tableting machine or a single-punch tableting machine. Furthermore, they have unexpectedly found that the preparation disintegrating quickly in the oral cavity manufactured as such has good disintegrability in the oral cavity and preferably shows good disintegrability even if no disintegrant other than the above-mentioned spray-dried powder comprising a sugar alcohol is specially added.

The present inventors have further conducted investigations and achieved the present invention.

Thus, the present invention relates to the followings.
(1) An intraorally rapidly disintegrable preparation, which comprises
   a spray-dried powder comprising a sugar alcohol, of which unit particle is a primary particle, and
   an active ingredient.
(2) The intraorally rapidly disintegrable preparation according to the above (1), wherein an average particle size of the unit particle is 5 to 150 µm.
(3) The intraorally rapidly disintegrable preparation according to the above (1) or (2), wherein the preparation further comprises a disintegrant.
(4) The intraorally rapidly disintegrable preparation according to the above (3), wherein the disintegrant is at least one member selected from the group consisting of crospovidone, crystalline cellulose·carmellose sodium, sodium carboxymethyl starch and hydroxypropyl starch.
(5) The intraorally rapidly disintegrable preparation d according to any of the above (1) to (4), wherein the sugar alcohol is at least one member selected from the group consisting of mannitol, erythritol, xylitol and sorbitol.
(6) The intraorally rapidly disintegrable preparation according to any of the above (1) to (5), wherein the spray-dried powder comprising a sugar alcohol, of which unit particle is a primary particle, the active ingredient and optionally the disintegrant are subjected to compression molding by the use of a rotary tableting machine or a single-punch tableting machine.
(7) The intraorally rapidly disintegrable preparation according to the above (6), wherein the compression molding is carried out by a direct tableting method.
(8) The intraorally rapidly disintegrable preparation according to the above (6) or (7), wherein the compression molding is carried out after a lubricant is previously applied to punches and a die of the tableting machine.
(9) A spray-dried powder comprising a sugar alcohol, of which unit particle is a primary particle.
(10) A process for producing the intraorally rapidly disintegrable preparation according to any of the above (1) to (5), which comprises compression-molding a spray-dried powder comprising a sugar alcohol, of which unit particle is a primary particle, an active ingredient and optionally a disintegrant by the use of a rotary tableting machine or a single-punch tableting machine.
(11) The process for producing the intraorally rapidly disintegrable preparation according to the above (10), wherein the compression molding is carried out by a direct tableting method.
(12) The process for producing the intraorally rapidly disintegrable preparation according to the above (10) or (11), wherein the compression molding is carried out after a lubricant is previously applied to punches and a die of the tableting machine.
(13) The spray-dried powder comprising a sugar alcohol according to the above (9), wherein an average particle size of the unit particle is 5 to 150 µm.
(14) The spray-dried powder comprising a sugar alcohol according to the above (9) or (13), wherein the sugar alcohol is at least one member selected from the group consisting of mannitol, erythritol, xylitol and sorbitol.
(15) The spray-dried powder comprising a sugar alcohol according to the above (9), (13) or (14), which comprises a saccharide and/or a binder.
(16) An additive for a preparation, which comprises the spray-dried powder according to any of the above (9), (13) to (15). In the present specification, the additive for a preparation is hereinafter just referred to as an additive.

With regard to the active ingredient contained in the intraorally rapidly disintegrable preparation of the present invention, there is no particular limitation so far as it is a physiologically active ingredient such as a pharmaceutically active ingredient and is used for oral administration. Further, it may be in any form such as a solid, a powder, a crystal, an oil, a solution or the like. In addition, food may be used as an active ingredient.

Specific examples of the physiologically active ingredient are one or more ingredient(s) selected from the group consisting of central nerve system drugs, peripheral nerve system drugs, drugs for circulation organs, drugs for respiratory organs, drugs for digestive organs, hormones, drugs for urogenital organs or anus, drugs for dental and oral use, vitamins, alimentary roborants, drugs for blood and body fluid, drugs for hepatic diseases, antidotes, drugs for habitual intoxication, gout treating agents, enzymes, antidiabetic agents, cell activation agents, anti-tumor agents, radioactive drugs, anti-allergy drugs, galenicals, antibiotics , chemotherapeutic agents, vaccines, anti-parasites drugs, diagnostic agents and narcotics/hallucinogens.

With regard to the active ingredient, it is also possible to use pharmacologically acceptable salts of the above-mentioned physiologically active ingredients, and examples thereof are salts with inorganic acids (e.g. hydrochloric acid, sulfuric acid and nitric acid), organic acids (e.g. succinic acid, acetic acid, propionic acid and trifluoroacetic acid), inorganic bases (e.g. alkaline metals such as sodium and potassium, and alkaline earth metals such as calcium and magnesium), organic basic compounds (e.g. organic amines such as triethylamine, and basic amino acids such as arginine), etc.

To be more specific, examples of the physiologically active ingredient contained in the intraorally rapidly disintegrable preparation according to the present invention are one or more component ( s ) selected from the group consisting of acetylspiramycin, amoxicillin, ethyl icosapentate, itraconazole, oxatomide, glybuzole, glutathione, ketophenylbutazone, cobamamide, cisapride, todralazine, tropisetron, domperidone, valproic acid, pyridoxal, fluorouracil, flunarizine, flurazepam, benidipine, minocycline, mebendazole, medroxyprogesterone, ubidecarenone, levodopa and pharmacologically acceptable salts thereof.

The active ingredient contained in the intraorally rapidly disintegrable preparation of the present invention may be coated by a known method *per se* for the purpose of masking taste and smell, being dissolved in intestine, releasing in a sustained manner, etc. Examples of the coating agent in this regard are an enteric polymer, an intragastically soluble polymer, a water-soluble polymer, a wax, etc.

The proportion of the active ingredient to be contained in the intraorally rapidly disintegrable preparation according to the present invention is about 0.005 to 60% by weight or, preferably, about 0.05 to 30% by weight.

Examples of the sugar alcohol used in the present invention are mannitol, erythritol, xylitol, sorbitol and pyranose derivatives and furanose derivatives thereof, and they may be used either solely or jointly. Among them, it is preferred to use mannitol from the viewpoint of its non-hygroscopicity, high melting point, good stability, no incompatibility with active ingredients, etc. With regard to the mannitol, more preferred one is D-mannitol.

The powder comprising sugar alcohol used in the present invention is a powder which is prepared by spray-drying and is characterized in that the unit particle of the sugar alcohol is a primary particle. As a result of the unit particle of the sugar alcohol being the primary particle as such, moldability of the resulting rapidly disintegrating preparation in the oral cavity is improved and, as a result, there is an advantage that hardness which is no problem in actual use is achieved. There is another advantage that, in spite of an increase in hardness, the disintegrability is improved as well.

The term "unit particle" means a minimum particle of spray-dried powder when it is dispersed in liquid in which the powder is insoluble (the spray-dried powder is not dissolved therein) or floated in the air. The expression "the unit particle is a primary particle" means that the unit particle is mostly primary particles and that, preferably not less than about 70% by weight, more preferably not less than about 80% by weight or, most preferably, not less than about 90% by weight of the unit particles are primary particles. Here, the term "primary particle" is a particle where, when microscopically checked, particles are not aggregated to assemble but each particle is in an independent state. In other words, in the spray-dried powder comprising a sugar alcohol used in the present invention, secondary particles which are formed by aggregation of the primary particles may be mixed in unit particles of the sugar alcohol.

An example of more preferred embodiments of the spray-dried powder comprising a sugar alcohol according to the present invention includes a spray-dried powder comprising a sugar alcohol, of which unit particle is a primary particle having an average particle size of about 5 to 150 µm.

An example of preferred process for producing the spray-dried powder comprising a sugar alcohol, of which unit particle is a primary particle will be illustrated below. Firstly, the sugar alcohol as mentioned above and, if necessary, other components (such as a saccharide, a binder, etc.) are completely dissolved in a solvent to prepare a solution of the sugar alcohol. With regard to the solvent, it is preferred to use water. Concentration of the sugar alcohol at that time is about 5 to 80% by weight or preferably about 10 to 45% by weight. When dissolving the sugar alcohol, it may be warmed, for example, at about 60 to 70°C.

After that, the solution of the sugar alcohol is spray-dried by a spray-drying granulator which is known *per se* (preferably, Spray Drier ML-12-BS-12 manufactured by Ohkawara Kakohki) or by a manufacturing machine having both spraying function and drying function such as a fluidized bed drying granulator, a tumbling fluidized bed granulator or a tablet coating machine, whereupon the powder is prepared. The condition for spray-drying in the above process is endothermic temperature of about 120 to 300°C or preferably about 150 to 220°C.

When two or more kinds of sugar alcohols are mixed and used in the intraorally rapidly disintegrable preparation according to the present invention, there may be used either a powder prepared by spray-drying a mixed solution where the two or more kinds of sugar alcohols are previously dissolved, or a mixed powder prepared by mixing spray-drying the two or more kinds of sugar alcohols after each of solutions of sugar alcohol is separately spray-dried.

The proportion of the spray-dried powder comprising the above sugar alcohol in the intraorally rapidly disintegrable preparation according to the present invention is about 30 to 99.9% by weight or preferably about 60 to 95% by weight.

The intraorally rapidly disintegrable preparation according to the present invention can be prepared from an active ingredient and the spray-dried powder comprising a sugar alcohol, of which unit particle is a primary particle, and is characterized by not needing to specifically add a disintegrant other than the spray-dried powder comprising the sugar alcohol. The fact that types of the additive is few is preferred in such respects that imbalance of bioavailability of active ingredients caused by additives is little and that analysis of the preparation can be carried out easily.

However, in the intraorally rapidly disintegrable preparation according to the present invention, a disintegrant may be contained besides the above spray-dried powder comprising the sugar alcohol, and such a preparation is one of preferred embodiments of the present invention.

Examples of the disintegrant used in the present invention are celluloses such as low substituted hydroxypropylcellulose and crystalline cellulose; starch or starch derivatives such as corn starch, partly pregelatinized starch and hydroxypropyl starch; crospovidone; bentonite and the like. More preferred examples are crospovidone, crystalline cellulose·carmellose sodium, sodium carboxymethyl starch and hydroxypropyl starch. The proportion of the disintegrant is preferably about 0.1 to 30% by weight or more preferably about 1 to 15% by weight. It is possible, by changing the proportion compounding amount, to prepare an intraorally rapidly disintegrable preparation having desired disintegrability and hardness.

Also the intraorally rapidly disintegrable preparation according to the present invention may contain other additives which are allowed to be used in preparations. To be more specific, there may be exemplified an excipient which is an inorganic salt such as calcium citrate or calcium phosphate; a lubricant such as magnesium stearate, calcium stearate, light anhydrous silicic acid or aqueous silicon dioxide; a surfactant such as sorbitan esters of fatty acid, polyoxyethylene esters of fatty acid, phospholipid, glycerol esters of fatty acid, polyethylene glycol esters of fatty acid, polyoxyethylene hydrogenated castor oil, polyoxyethylene alkyl ether or sucrose esters of fatty acid; a foaming agent such as sodium bicarbonate, sodium carbonate, calcium carbonate or calcium bicarbonate; a solubilizing agent such as cyclodextrin, arginine, lysine or trisaminomethane; an organic acid such as citric acid, tartaric acid or malic acid; a color such as iron sesquioxide, yellow iron sesquioxide or tar dye; a flavor such as lemon, lemon lime, orange, pineapple, mint, menthol or camphor; a sweeten agent such as saccharin, glycyrrhizinic acid, aspartame, stevia or thaumatin; a corrigent such as citric acid, sodium citrate, succinic acid, tartaric acid, fumaric acid or glutamic acid; or the like.

It is also possible to mix a saccharide such as lactose, sucrose, glucose or trehalose in the intraorally rapidly disintegrable preparation according to the present invention, and it is further possible to mix a binder such as hydroxypropyl cellulose, hydroxypropyl methylcellulose, pregelatinized starch, polyvinyl alcohol, polyvinylpyrrolidone, acacia, gelatin or pullulan. Those saccharide and binder may also be mixed with a solution of a sugar alcohol at the time when the solution of a sugar alcohol is spray-dried.

Thus, the present invention relates to an intraorally rapidly disintegrable preparation, which comprises a spray-dried powder of a sugar alcohol, of which the unit particle is a primary particle, and an active ingredient, and the spray-dried powder may further contain components other than a sugar alcohol such as a saccharide and a binder.

The spray-dried powder of a sugar alcohol may be used as an additive as it is or may also be used for producing the intraorally rapidly disintegrable preparation after the powder is made into an additive of a premixed type with other additives or after the powder is made into a bulk type additive granulated or compression-molded with other additives, and they are also one of the preferred embodiments of the present invention.

In the intraorally rapidly disintegrable preparation according to the present invention, there is no particular limitation for the proportion of an additive, and it may be the amount which has been usually used in the related art.

Manufacture of the intraorally rapidly disintegrable preparation according to the present invention may be carried out by a known method *per se* for producing compression-molded preparations such as tablets using an active ingredient, spray-dried powder comprising the above-mentioned sugar alcohol, of which unit particle is primary particle and optionally an additive such as a disintegrant.

To be more specific, there may be exemplified an indirect tableting method where an active ingredient, spray-dried powder comprising the above-mentioned sugar alcohol and optionally an additive such as a disintegrant are previously granulated by a granulator known *per se* such as a fluidized bed granulator, a stirring granulator, a tumbling granulator, a tumbling fluidized bed granulator, an extrusion granulator and a dry granulator, then mixed with an additive such as a lubricant, and subjected to a tableting machine known *per se,* preferably a rotary tableting machine with good productivity or a single-punch tableting machine.

Another example is a direct tableting method where an active ingredient, spray-dried powder comprising the above-mentioned sugar alcohol, of which unit particle is primary particle and optionally an additive such as a disintegrant are mixed, and the mixture is subjected to a tableting machine known *per se,* preferably a rotary tableting machine with good productivity or a single-punch tableting machine.

In any of the above-mentioned methods, it is preferred that the pressure for compression molding is not less than about 5,000 N.

In the manufacture of the intraorally rapidly disintegrable preparation according to the present invention, any of the above-mentioned indirect tableting methods or direct tableting methods may be used. However the direct tableting methods are easier and more convenient as compared with the indirect tableting methods and, in addition, they do not cause problems in stability, etc. during granulation. Accordingly, it is preferred to adopt the direct tableting methods.

In any of the manufacturing methods according to the indirect tableting methods and the direct tableting methods, it is possible that, during the compression molding, compression molding can be carried out by previously applying a lubricant to punch and die of the tableting machine, without mixing a lubricant with the granule in the indirect tableting method or with the mixed powder in the direct tableting method, whereby the effect of the invention is enhanced.

There is no particular limitation for the shape of the intraorally rapidly disintegrable preparation prepared by the present invention, and the shape may be a round shape or various different shapes having, for example, a normal round surface, a sugar-coated round surface, a sharp-cornered plane, a round-cornered plane, a two-layered round surface, etc. The preparation may also be used as a dividable tablet having groove. It may further be a multi-layered tablet having two or more layers.

The "intraorally rapidly disintegrable preparation" according to the present invention is a preparation which easily disintegrates or dissolves in the oral cavity as mentioned above. In the present invention, the preferred one is a compression-molded preparation. The "intraorally rapidly disintegrable preparation" according to the present invention may further cover granules which are prepared by pulverizing or crashing the above-prepared compression-molded preparation.

Such a preparation has advantages that it can be taken even under such a state where it is unable to drink water easily during, for example, traveling, working or riding on a vehicle. In addition, since there is no worry of such inconvenience as occurs upon taking a drinkable ampoule, etc. (e.g. spilling or dripping) and no big space is necessary for carrying, it is suitable as a "portable" pharmaceutical form.

The preparation according to the present invention is also useful when small children or aged people need to take an active ingredient. In other words, even the above-mentioned people having difficulty in swallowing the conventional tablets and capsules, i.e. those who are unable to swallow the conventional tablets and capsules in the oral are now able to easily take them. Also, the preparation of the present invention has a high safety that, for example, they are less likely to get caught in the throat as compared with the conventional tablets or capsules. Further advantages are that, unlike syrups, there is no troublesome work of measuring the amount when taking, and an error in the dose hardly takes place.

The intraorally rapidly disintegrable preparation according to the present invention is characterized by having hardness which is no problem in practical use and showing good disintegrability in the oral cavity. To be more specific, as an index for good disintegrability, appropriate time for disintegration in the oral cavity is about 5 to about 120 seconds, preferably about 5 to about 60 seconds or, more preferably, about 5 to about 20 seconds. The time for disintegration in the oral cavity means the time requiring for a solid preparation being completely disintegrated by saliva in the oral cavity of healthy male and female adults.

Further, as an index for having hardness which is no problem in practical use, appropriate hardness of the preparation is about 30 to 150 N. The hardness can be easily measured by a tablet hardness tester which is known *per se.*

In the present invention, the spray-dried power comprising a sugar alcohol can become an additive when it is used during the manufacture of a preparation such as an intraorally rapidly disintegrable preparation.

### Best Mode for Carrying Out the Invention

The present invention will be further illustrated by way of the following Example and Comparative Example, although they do not limit the present invention.

### Example 1

D-Mannitol (Towa chemical Industry; average particle size being about 60 µm) was dissolved in water to prepare a solution wherein the concentration of D-mannitol was 15% by weight. The solution was spray-dried by a spray-drier (manufactured by Ohkawara Kakohki; Spray Drier ML-12-BS-12) to give spray-dried powder of D-mannitol. Endothermic temperature during the spray drying was 200°C. When the spray-dried powder was observed under a microscope, it was found to consist of primary particles of average particle size of about 50 µm.

The D-mannitol (1820 g), 100 g of crospovidone (GAF; Polyplasdone XL-10), 40 g of magnesium stearate and 40 g of benidipine hydrochloride were mixed. The mixture was made into tablets using a rotary tableting machine (manufactured by Hata Seisakusho; type AP-18) to give an intraorally rapidly disintegrable preparation. The tableting conditions for manufacturing were that weight per a tablet was 200 mg, the metal mold was a round-shape one with sharp edge having 8 mm diameter and compression pressures were 6000, 9000 and 12000 N.

### Comparative Example 1

D-Mannitol (Towa chemical Industry; average particle size being about 60 µm) (1820 g), 100 g of crospovidone (GAF; Polyplasdone XL-10), 30 g of magnesium stearate and 40 g of benidipine hydrochloride were mixed. The mixture was made into tablets using a rotary tableting machine (manufactured by Kikusui Seisakusho; type Collect 12) to give the preparation. The tableting conditions were the same as those in Example 1.

### Test Example

Hardness and disintegration time of the preparations prepared in Example 1 and Comparative Example 1 were measured by the following methods. Hardness was measured by a tablet hardness tester (manufactured by Japan Machinery; type PTB-311) and average strength of ten preparations was defined as the hardness of each preparation. With regard to the disintegration time, a disintegration test was carried out according to the Japanese Pharmacopoeia XIII and average disintegration time was defined as the disintegration time for each preparation.

The results are shown in the following Table.

**Table 1**

| Sample/Compression Pressure | | 6000 N | 9000 N | 12000 N |
|---|---|---|---|---|
| Example 1 | Hardness | 37.1 N | 57.0 N | 70.2 N |
| | Disintegration time | 19.1 sec | 19.7 sec | 22.7 sec |
| Comparative Example 1 | Hardness | Tableting Impossible | 28.0 N | Tableting Impossible |
| | Disintegration time | Tableting Impossible | 20.4 sec | Tableting Impossible |

In Example 1 where there was used spray-dried powder of a sugar alcohol, of which unit particle was a primary particle, it was possible to give an intraorally rapidly disintegrable preparation with sufficient hardness having no problem in practical use and rapid disintegration, while in Comparative Example 1 where there was used a sugar alcohol powder which was not obtained by spray-drying, the tableting was impossible or, even when tableting was possible and rapid disintegration was achieved, it was not possible to obtain a preparation satisfying a sufficient hardness having no problem in practical use.

### Industrial Applicability

In accordance with the present invention, it is possible to manufacture a preparation using a tableting machine which is known *per se* or, preferably, a rotary tableting machine having good productivity or a single-punch tableting machine by a common compression molding which is generally used in the art or, preferably, a direct tableting method, and it is also possible to provide an intraorally rapidly disintegrable preparation having hardness without a problem in practical use and showing rapid disintegration.

Use of the spray-dried powder comprising a sugar alcohol, of which unit particle is a primary particle according to the present invention as an additive for an intraorally rapidly disintegrable preparation is epoch-making in view of improvement of disintegrability and of maintenance of hardness having no problem in practical use.

## Claims

1. An intraorally rapidly disintegrable preparation, which comprises
a spray-dried powder comprising a sugar alcohol, of which unit particle is a primary particle, and
an active ingredient.

2. The intraorally rapidly disintegrable preparation according to claim 1, wherein an average particle size of the unit particle is 5 to 150 µm.

3. The intraorally rapidly disintegrable preparation according to claim 1 or 2, wherein the preparation further comprises a disintegrant.

4. The intraorally rapidly disintegrable preparation according to claim 3, wherein the disintegrant is at least one member selected from the group consisting of crospovidone, crystalline cellulose·carmellose sodium, sodium carboxymethyl starch and hydroxypropyl starch.

5. The intraorally rapidly disintegrable preparation according to any one of claims 1 to 4, wherein the sugar alcohol is at least one member selected from the group consisting of mannitol, erythritol, xylitol and sorbitol.

6. The intraorally rapidly disintegrable preparation according to any one of claims 1 to 5, wherein the spray-dried powder comprising a sugar alcohol, of which unit particle is a primary particle, the active ingredient and optionally the disintegrant are subjected to compression molding by the use of a rotary tableting machine or a single-punch tableting machine.

7. The intraorally rapidly disintegrable preparation according to claim 6, wherein the compression molding is carried out by a direct tableting method.

8. The intraorally rapidly disintegrable preparation according to claim 6 or 7, wherein the compression molding is carried out after a lubricant is previously applied to punches and a die of the tableting machine.

9. A spray-dried powder comprising a sugar alcohol, of which unit particle is a primary particle.

10. A process for producing the intraorally rapidly disintegrable preparation according to any one of claims 1 to 5, which comprises compression-molding a spray-dried powder comprising a sugar alcohol, of which unit particle is a primary particle, an active ingredient and optionally a disintegrant by the use of a rotary tableting machine or a single-punch tableting machine.

11. The process for producing the intraorally rapidly disintegrable preparation according to claim 10, wherein the compression molding is carried out by a direct tableting method.

12. The process for producing the intraorally rapidly disintegrable preparation according to claim 10 or 11, wherein the compression molding is carried out after a lubricant is previously applied to punches and a die of the tableting machine.

13. The spray-dried powder comprising a sugar alcohol according to claim 9, wherein an average particle size of the unit particle is 5 to 150 µm.

14. The spray-dried powder comprising a sugar alcohol according to claim 9 or 13, wherein the sugar alcohol is at least one member selected from the group consisting of mannitol, erythritol, xylitol and sorbitol.

15. The spray-dried powder comprising a sugar alcohol according to claim 9, 13 or 14, which comprises a saccharide and/or a binder.

16. An additive for a preparation, which comprises the spray-dried powder according to any one of claims 9, 13 to 15.
